Europäisches Patentamt

European Patent Office

Office européen des brevets

(19)

(11) Publication number: **0 099 634 B1**

# (12) EUROPEAN PATENT SPECIFICATION

(45) Date of publication of patent specification: **07.01.87**

(51) Int. Cl.⁴: **C 12 M 1/08, B 01 J 19/24**

(21) Application number: **83303242.8**

(22) Date of filing: **06.06.83**

(54) Reactor apparatus for multiphase contacting.

(30) Priority: **07.06.82 US 386052**
**10.06.82 GB 8216858**

(43) Date of publication of application:
**01.02.84 Bulletin 84/05**

(45) Publication of the grant of the patent:
**07.01.87 Bulletin 87/02**

(84) Designated Contracting States:
**DE FR GB IT**

(56) References cited:
**CH-A- 472 496**
**CH-A- 578 887**
**FR-A- 958 486**
**FR-A-1 373 751**
**US-A-4 036 699**

(73) Proprietor: **UNIVERSITY OF WATERLOO**
**Waterloo Ontario (CA)**

(72) Inventor: **Young, Murray Moo**
**50 Bluesprings Drive Apt. 63**
**Waterloo Ontario N2J 4M1 (CA)**

(74) Representative: **Silverman, Warren et al**
**HASELTINE LAKE & CO. Hazlitt House 28**
**Southampton Buildings Chancery Lane**
**London WC2A 1AT (GB)**

Courier Press, Leamington Spa, England.

## Description

The present invention relates to apparatus and method for carrying out multiphase contacting in liquids for the promotion of physical, chemical or biological changes, more particularly bioconversion and biotransformation processes, as in fermentation and enzymatic processes, utilizing such apparatus.

Particulate solids, such as, microbial, animal or plant cells or their components attached to artificial carriers, or nutrient substrates, are involved in many useful and potentially useful bioconversion or biotransformation processes. In carrying out such processes, it is often essential to contact the solid particles with a liquid phase to permit biochemical reactions involving components contained in the solids and in the liquid phase to occur. It also may be essential for a gas solute, for example, oxygen, to be transferred from a gas phase, such as, air, into the liquid medium. In addition, the processes may need to be carried out under mild conditions of mixing and agitation in order to avoid deleterious effects, such as, cellular damage or excessive use of energy for mixing and/or aeration.

In the prior art, such bioconversion or biotransformation processes are accomplished in reactor devices which normally employ simple mechanically-stirred or pneumatically-stirred vessels. These prior art devices, however, suffer from one or more of several drawbacks, including the accumulation of particulate solids in unmixed stagnant zones in the vessel, the growth of organisms in crevices and other stagnant-zone or poorly mixed regions of the vessel, the development of foam at the top of the aerated medium, and damage and destruction of biological cells by higher shear and excessive use of energy for mixing and/or aeration.

In accordance with the present invention, these prior art problems are overcome by effecting the procedures in a uniquely-constructed vessel. The apparatus of the invention enables there to be achieved solids deposit-free bioconversion and/or foam free bioconversion and/or mild agitation bioconversions, as well as other physical and/or chemical reactions.

In accordance with the present invention, there is provided, in one aspect a method of effecting multiphase contacting between gas, solid and liquid phases, comprising the steps of:

(a) establishing a continuous liquid phase in which at least one particulate solid phase is suspended in a cylindrical upright mixing zone having a height-to-diameter ratio of 1:1 to 4:1 and a lower end closure having a perimetrical surface, said lower end closure comprising an upwardly-extending conical surface having a solid angle of 30° to 90° with the perimetrical surface being located between the perimeter of the conical surface and the inner wall of the mixing zone;

(b) establishing concentric coaxial flow paths within the mixing zone which are maintained physically separate from one another but in fluid flow relationship with one another at the upper and lower ends thereof, the ratio of the diameter of the mixing zone to the diameter of the inner flow path being from 2:1 to 5:1; and

(c) feeding at least one gas in bubble form from the perimetrical surface into the outer one of the coaxial flow paths to cause the continuous liquid phase first to flow upwardly therein, then to flow downwardly through the inner one of the coaxial flow paths towards the lower end closure and to flow back into the outer flow path while maintaining the solid phase in suspension. As will be explained in greater detail hereinafter, the method of this invention ensures the absence of stagnant zones within the upright vessel and the avoidance of zones of high shear.

In a second aspect, this invention provides an apparatus for effecting multiphase contacting between gas, solid and liquid phases, wherein

(a) an upright cylindrical vessel having height-to-diameter ratio of 1:1 to 4:1;

(b) a conical surface having a solid angle of 30° to 90° extending upwardly within the vessel from the lower end and having its perimeter spaced inwardly from the vessel wall;

(c) a gas sparger located at the lower end of the vessel and in the gap between the inner wall and the conical surface perimeter for admitting at least one gas in bubble form into a continuous liquid phase in which particulate solid phase is suspended contained in the vessel; and

(d) a cylindrical draft tube located within the vessel coaxial therewith with its lower end spaced upwardly from the apex of the conical surface and its upper end spaced downwardly from the intended liquid level in the vessel, the ratio of the diameter of the vessel to the diameter of the draft tube being from 2:1 to 5:1.

This invention is described further, by way of illustration, with reference to the accompanying drawings, in which:

FIGURE 1 is a schematic elevational sectional view of a reactor apparatus provided in accordance with one embodiment of the invention; and

FIGURE 2 is a plan view of the reactor apparatus of Figure 1.

Referring to the drawings, which illustrate the current best mode of carrying out the invention known to the applicant, an upright generally right-cylindrical reactor vessel 10 houses a liquid phase medium 12 which contains solid particulates 14. The solid particulates 14 may be any substance which contains one or more components capable of undergoing physical, chemical or biochemical reactions with components of the liquid medium 12. Such substances include solid cellulosic substrates, metal-bearing ores, cells of microbes, plants or animals, or artificially-immobilized enzymes.

The liquid medium 12 may contain one or more components capable of undergoing chemical or biochemical reactions with components of the solid particulates 14. The liquid phase reaction medium, for example, may comprise an aqueous nutrient medium for fermentation containing dissolved salts, sugars and other components commonly used in fermentation media.

The reactor vessel 10 has a height-to-diameter ratio in the range of from 1:1 to 4:1, preferably about 3:1.

A gas sparger 16 for the introduction, of at least one gas, takes the form of an array of orifices 17 located around the outer perimeter of the base 18 of the reactor vessel 10. The orifices 17 may have any convenient diameter, generally from 0.05 to 1 cm, typically about 0.3 cm, and may be spaced apart any convenient distance, usually from 1 to 10 cm, typically about 1.25 cm. Any other convenient form of gas sparger 16, such as, a perforated hollow ring, may be provided. Additional aeration and/or agitation may be effected, if desired, by the use of two or more such gas spargers 16.

An inverted cone 20 forms the bottom wall 18 of the reactor vessel 10 and its perimeter is spaced from the upright wall 22 of the reactor 10, so that the orifices 17 of the gas sparger 16 communicate with the crevice 23 which is formed by the bottom and side walls 18, 22 of the reactor 10 and the inverted cone 20. The inverted cone 20 has a conical angle a of from 30° to 90°.

The gas sparger 16 injects gas bubbles 24 through the multiple orifices 17 into the crevice 23 and thereby into the liquid medium 12.

'A right cylindrical draft tube 26 is arranged coaxially with the reactor vessel 10 and is located within the liquid medium spaced upwardly from the inverted cone 20, inwardly from the reactor side wall 22 and downwardly from the surface of the liquid medium 12 in the reactor 10.

The draft tube 26 has a diameter such that the ratio of the diameter of the vessel 10 to the diameter of draft tube 26 is from 2:1 to 5:1, preferably about 4:1. The draft tube 26 may be spaced upwardly from the inverted cone 20 and downwardly from the upper level of liquid medium 12 any convenient distance, typically about the same distance. Generally, the distance is about 5 to about 10 cm.

The draft tube 26 separates the interior of the reactor vessel 10 into concentric flow channels 28 and 30 through which the reaction medium 12 flows in opposite directions. By the nature of the operations which are effected in the reactor vessel 10, the flow channels 26 and 28 are formed even if the draft tube is omitted. The draft tube 26, however, is usually used to provide a clear demarkation between the flow channels and prevents cross-mixing.

An axial downflow-type impeller 32, for example, a propeller, may be located within the flow channel 30 to assist in the suppression of foam formation and a supplementary ring sparger 34 may be located between the outer wall of the draft tube 26 and the inside wall of the reactor vessel 10 to increase the gas flow to the liquid medium 12. A heat exchanger (not shown) also may be associated with the draft tube 26 to enable the liquid medium 12 to be heated or cooled, as required.

In operation, a gas, usually air where an aerobic reaction is required, or an inert gas where an anaerobic reaction is required, is fed into the reactor vessel 10 through the gas sparger 16, thereby forming the gas bubbles 34 in the crevices 23. The gas bubbles 24 rise in the crevices 23, thereby clearing and cleaning the crevices 23 of any particulate solids which may settle therein.

The gas bubbles pass into the outer annular flow channel 28 and create a hydrostatic pressure difference between the contents of the annular flow channel 28 and the inner flow channel 30. A suction effect is created within the inner flow channel 30, thereby causing the reaction medium 12 in the outer annular channel 28 to flow upwardly until it clears the upper edge of the draft tube 26, to flow downwardly through the inner channel 30 until it clears the bottom edge of the draft tube 26, and then to flow once again into the annular channel 28.

The suction effect created within the inner flow channel 30 may be enhanced by the action of the impeller 32. The impeller 32 also assists in liquid recirculation within the reactor 10. More than one such impeller 32 may be utilized, depending on the vertical height of the vessel and the intensity of the recirculation required.

The suction effect in the inner flow channel 30 carries any undesirable foam which may form on the surface of the reaction medium 12 in the reactor 10 into the bulk liquid below the surface where the foam is destroyed by re-dissolution and/or bubble coalescence.

Additional aeration and/or agitation may also be effected, if desired, by the utilization of one or more supplementary ring spargers 34. The orifices of this sparger 34 are arranged to eject gas radially outwardly thereof so that small preformed bubbles are readily detached by the upward fluid flow in the channel 28.

The above description of the preferred embodiment of the invention is with respect to a right cylindrical reactor vessel 10 having a coaxial right cylindrical draft tube 26. The broadest scope of the invention, however, permits the use of alternative geometrical configurations to which the term cylinder used herein extends. For example, the reactor vessel 10 may have a square or rectangular cross section, while the draft tube 26 may be replaced by a conduit of square or rectangular cross section, while the draft rectangular cross section. In an alternative structure, the draft tube 26 may be formed by two flat baffle plates which extend between the sides of the vessel.

The inverted insert 20 is illustrated as having a conical shape and this term is intended herein to extend to a pyramid shape. The insert 20 may be integrally formed with the reactor vessel 10 to constitute the bottom wall 18, rather than as the separate member illustrated. The gas sparger 34 generally follows the geometrical shape of the draft tube 26.

The reactor vessel 10 may be used for carrying out a wide variety of bioconversion and biotransformation processes in a manner which overcomes the prior art problems of mechanically stirred vessels. The entry of the gas to the reactor vessel 10 and the through and uniform mixing of the solid phase and liquid phase within the reactor vessel 10 prevents the accumulation of particulate solids in unmixed stagnant zones and the growth of cellular biomass in crevices and other stagnant zones or poorly mixed regions of the reactor vessel 10. The induced suction in the channel 30 eliminates foam accumulation on the surface of the liquid medium 12 and produces only low shear forces on biological cells, so that damage or destruction of the cells by shear forces is eliminated. In this way, considerably enhanced microbial growth can be achieved using this invention.

The bioconversion or biotransformation process which is effected using the apparatus of the invention may be a fermentation process using any desired organism, for example, aerobic fermentation using *Chaetomium cellulolyticum* (American Type Culture Collection, Rockville, Maryland, Accession No. 32319).

Where the latter organism is used for the fermentation, the particulate solid phase material 14 usually is a cellulosic-containing material to provide carbon nutrient for the organism growth. The cellulosic-containing material may be any convenient cellulosic material, such as, wheat straw, corn stover or Kraft paper pulp mill clarifier waste sludge. The cellulosic material usually is in the form of particulate solids of up to about 2 cm particle mesh size.

The cellulosic material may be provided in any convenient concentration for carrying out the fermentation, usually up to about 5% w/v (weight per unit volume).

The flow rate of gas through the sparger 16 into the reactor 10 may be any convenient value, depending on the process which is being effected in the reactor vessel 10. Usually, the gas flow rate is in the range of up to about 2 volumes of gas per volume of liquid medium per minute.

Gas-liquid mass transfer is achieved in the vessel 10 at rates comparable to conventional high shear mechanically-stirred devices. Further, the efficiency of liquid mixing within the vessel 10 is superior to conventional low-shear pneumatically-stirred devices.

The invention is illustrated by the following Examples:

### Example 1

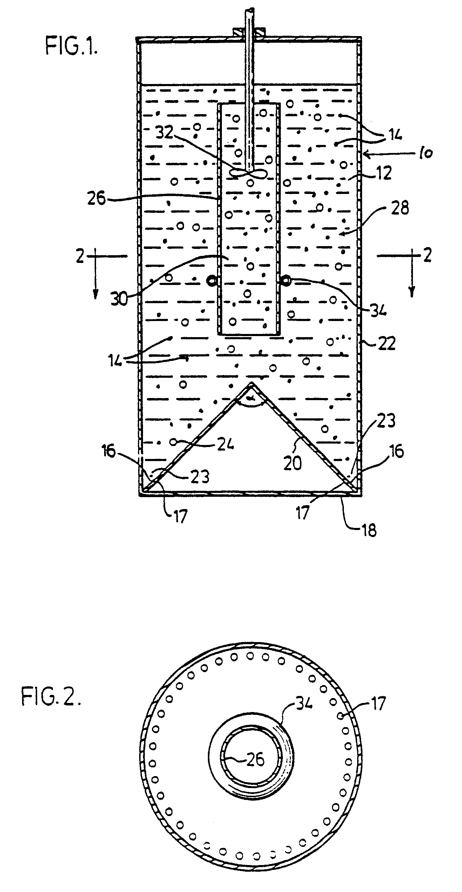

Using the apparatus of Figure 1 having a height-to-diameter ratio of 3, microbial biomass in the form of the fungal organism, *Chaetomium cellulolyticum*, was produced by growing it in an aerated aqueous nutrient medium containing 1% particulate solids in the form of chemically pretreated pieces of wheat straw (average mesh size of 2 mm) as the sole carbon source for the fermentation process. The process proceeded with virtually no interference from microbial growth attachments to the vessel walls or bottom or settling out of particulate solids within the device.

By contrast, undesirable cellular disruptions, wall attachments and settling-out of solids leading to about 25% lesser microbial biomass production (of a maximum of about 0.2 grams per litre of medium per day) within the vessel medium occurred for similar experiments carried out in a conventional mechanically stirred tank fermentor of equivalent volume and aeration rates.

### Example 2

Repeating the set of comparative experiments in Example 1 but replacing the wheat straw with corn stover, similar better results of microbial biomass productivity (by about 25%) were obtained with the apparatus of Figure 1.

### Example 3

Repeating the set of comparative experiments in Example 1 but replacing the wheat straw with a Kraft paper pulp mill clarifier waste sludge similar better results of microbial biomass productivity (by about 25%) were obtained with the apparatus of Figure 1.

### Example 4

Using the apparatus of Figure 1, the rate of oxygen transfer from sparged air bubbles into the liquid phase of a typical fermentation medium was measured as an overall mass transfer coefficient of about 120 reciprocal hours. It was found that the oxygen transfer efficiency in terms of power requirements per unit volume of vessel contents was comparable to that obtained in a conventional high-shear mechanically stirred tank fermentor device of the same size and for the same aeration conditions.

### Example 5

Using the apparatus of Figure 1, the volume of air bubbles retained in the liquid phase of a typical fermentation medium was measured as 12% volume of gas per volume of liquid. It was found that a similar gas-bubble retention was obtained in a conventional low shear bubble column of the same size and for the same aeration conditions.

### Example 6

Repeating the set of comparative experiments in Example 4 but measuring liquid blending time instead of oxygen transfer rates, it was found that the apparatus of Figure 1 was superior by 10 to 50% to the conventional bubble column fermentor device for comparable aeration rates.

In summary of this disclosure, the present invention provides novel apparatus having improved operating parameters in physical, chemical or biochemical processes, including bioconversion and biotransformation processes.

**Claims**

1. A method of effecting multiphase contacting between gas, solid and liquid phases, characterized by the steps of:

(a) establishing a continuous liquid phase in which at least one particulate solid phase is suspended in a cylindrical upright mixing zone having a height-to-diameter ratio of 1:1 to 4:1 and a lower end closure having a perimetrical surface, said lower end closure comprising an upwardly-extending conical surface having a solid angle of 30° to 90° with the perimetrical surface being located between the perimeter of the conical surface and the inner wall of the mixing zone;

(b) establishing concentric coaxial flow paths within the mixing zone which are maintained physically separate from one another but in fluid flow relationship with one another at the upper and lower ends thereof, the ratio of the diameter of the mixing zone to the diameter of the inner flow path being from 2:1 to 5:1, and

(c) feeding at least one gas in bubble form from the perimetrical surface into the outer one of the coaxial flow paths to cause the continuous liquid phase first to flow upwardly therein, then to flow downwardly through the inner one of the coaxial flow paths towards the lower end closure and to flow back into the outer flow path while maintaining the solid phase in suspension.

2. The method claimed in claim 1, characterized in that at least one additional gas in bubble form is fed into the outer flow path transversely outwardly thereof.

3. The method claimed in claim 1 or 2, characterized in that the continuous liquid phase is subjected to an auxiliary impeller force downwardly within the inner flow path.

4. The method claimed in any one of claims 1 to 3, characterized in that the gas comprises air, the continuous liquid phase comprises a fermentation medium, and the particulate solids comprise an organism and a solid substrate for the organism.

5. The method claimed in claim 4, characterized in that the particulate solids comprise a cellulosic-containing material and the organism is a cellulolytic organism.

6. An apparatus for effecting multiphase contacting between gas, solid and liquid phases, characterized by

(a) an upright cylindrical vessel having height-to-diameter ratio of 1:1 to 4:1,

(b) a conical surface having a solid angle of 30° to 90° extending upwardly within the vessel from the lower end and having its perimeter spaced inwardly from the vessel wall;

(c) a gas sparger located at the lower end of the vessel and in the gap between the inner wall and the conical surface perimeter for admitting at least one gas in bubble form into a continuous liquid phase in which particulate solid phase is suspended contained in the vessel; and

(d) a cylindrical draft tube located within the vessel coaxial therewith with its lower end spaced upwardly from the apex of the conical surface and its upper end spaced downwardly from the intended liquid level in the vessel, the ratio of the diameter of the vessel to the diameter of the draft tube being from 2:1 to 5:1.

7. The apparatus claimed in claim 6, characterized in that the gas sparger comprises a plurality of orifices of diameter 0.05 to 1 cm formed through the lower closure adjacent the vessel wall and equally circumferentially spaced apart 1 to 10 cm.

8. The apparatus claimed in claim 6 or 7, characterized in that the draft tube is located 5 to 10 cm above the apex of the conical surface and 5 to 10 cm below the intended liquid level in the vessel.

9. The apparatus claimed in any one of claims 6 to 8, characterized in that an auxiliary gas sparger surrounds the draft tube and is constructed to eject gas in bubble form radially outwardly thereof into the liquid phase.

10. The apparatus claimed in any one of claims 6 to 9, characterized in that a liquid downflow impeller is located adjacent the upper end of the draft tube.

## Patentansprüche

1. Verfahren zum Herstellen eines Mehrphasenkontaktes zwischen Gas-, Feststoff- und Flüssigkeitsphasen, gekennzeichnet durch die folgenden Schritte:

(a) die Einrichtung einer kontinuierlichen Flüssigkeitsphase, in der mindestens eine aus Partikeln bestehende Feststoffphase in einer zylindrischen aufrechten Mischzone mit einem Verhältnis von Höhe zu Durchmesser von 1:1 bis 4:1 und einem unteren Endabschluß mit einer perimetrischen Fläche suspendiert ist, wobei der untere Endabschluß eine sich nach oben erstreckende konische Oberfläche aufweist, die mit der zwischen dem Umfang der konischen Oberfläche und der Innenwand der Mischzone gelegenen perimetrischen Fläche einen Raumwinkel von 30° bis 90° bildet;

(b) die Einrichtung konzentrischer koaxialer Fließwege innerhalb der Mischzone, die physikalisch voneinander getrennt gehalten werden, aber an den oberen und unteren Enden der Fließwege in Fließbeziehung miteinander gehalten werden, wobei das Verhältnis vom Durchmesser der Mischzone zum Durchmesser des inneren Fließweges 2:1 bis 5:1 beträgt; und

(c) das Zuführen mindestens eines Gases in Blasenform von der perimetrischen Fläche in den äußeren der koaxialen Fließwege, um zuerst ein Aufwärtsfließen der kontinuierlichen Flüssigkeitsphase in diesem, anschliessend deren Abwärtsfließen durch den inneren der koaxialen Fließwege zum unteren Endabschluß und deren Rückfließen in den äußeren Fließweg zu bewirken, während die Feststoffphase suspendiert gehalten wird.

2. Verfahren nach Anspruch 1, dadurch gekennzeichnet, daß mindestens ein zusätzliches Gas in Blasenform in den äußeren Fließweg im Verhältnis zu diesem quer nach außen gerichtet eingeführt wird.

3. Verfahren nach Anspruch 1 oder 2, dadurch gekennzeichnet, daß die kontinuierliche Flüssigkeitsphase im inneren Fließweg einer abwärts gerichteten Hilfs-Antriebskraft ausgesetzt wird.

4. Verfahren nach einem der Ansprüche 1 bis 3, dadurch gekennzeichnet, daß das Gas Luft enthält, die kontinuierliche Flüssigkeitsphase ein Fermentierungsmittel enthält und die Feststoffpartikel einen Organismus und ein festes Substrat für den Organismus enthalten.

5. Verfahren nach Anspruch 4, dadurch gekennzeichnet, daß die Feststoffpartikel ein zellulosehaltiges Material enthalten und der Organismus ein zellulolytischer Organismus ist.

6. Vorrichtung für Mehrphasenkontakte zwischen Gas-, Feststoff- und Flüssigkeitsphasen, gekennzeichnet durch

(a) einen aufrechtstehenden zylindrischen Behälter mit einem Verhältnis von Höhe zu Durchmesser von 1:1 bis 4:1,

(b) eine konische Oberfläche mit einem Raumwinkel von 30° bis 90°, die sich innerhalb des Behälters von deren unteren Ende aufwärts erstreckt und deren Umfang einen Abstand von der Wand das Behälters nach innen hin besitzt;

(c) einen am unteren Ende des Behälters und in der Spalte zwischen der Innenwand und dem Umfang der konischen Oberfläche angebrachten Gasverteiler zum Einführen mindestens eines Gases in Blasenform in eine in dem Behälter enthaltene kontinuierliche Flüssigkeitsphase, in der eine aus Partikeln bestehende Feststoffphase suspendiert ist; und

(d) ein innerhalb des Behälters koaxial zu diesem angebrachtes zylindrisches Strömungsrohr, dessen unteres Ende von der Spitze der konischen Oberfläche nach oben einen Abstand aufweist und deren oberes Ende vom vorgesehenen Flüssigkeitspegel im Behälter nach unten einen Abstand aufweist, wobei das Verhältnis vom Durchmesser des Behälters zum Durchmesser des Strömungsrohrs 2:1 bis 5:1 beträgt.

7. Vorrichtung nach Anspruch 6, dadurch gekennzeichnet, daß der Gasverteiler mehrere Austrittsöffnungen mit einem Durchmesser von 0,05 bis 1 cm aufweist, die durch den unteren Endabschluß an die Behälterwand angrenzend gebildet sind und die umfangsmäßig mit einem gleichen Abstand von 1 bis 10 cm angebracht sind.

8. Vorrichtung nach Ansruch 6 oder 7, dadurch gekennzeichnet, daß das Strömungsrohr 5 bis 10 cm über der Spitze der konischen Oberfläche und 5 bis 10 cm unter dem vorgesehenen Flüssigkeitspegel in dem Behälter angebracht ist.

9. Vorrichtung nach einem der Ansprüche 6 bis 8, dadurch gekennzeichnet, daß ein Hilfs-Gasverteiler das Strömungsrohr umgibt und so ausgebildet ist, daß er Gas in Blasenform radial nach außen von diesem in die Flüssigkeitsphase ausstößt.

10. Vorrichtung nach einem der Ansprüche 6 bis 9, dadurch gekennzeichnet, daß ein Flüssigkeitsabstrom-Antrieb nahe am oberen Ende des Strömungsrohrs angebracht ist.

**Revendications**

1. Procédé pour effectuer un contact multiphases entre des phases gazeuses, solides et liquides, caractérisé par les étapes suivantes:

(a) établissement d'une phase liquide continue dans laquelle au moins une phase solide formée de particules est en suspension dans une zone de mélange verticale et cylindrique ayant une rapport de hauteur à diamètre de 1:1 à 4:1 et comportant un élément de fermeture d'extrémité inférieure comprenant une surface périphérique, ledit élément de fermeture d'extrémité inférieure comprenant une surface conique s'étendant vers le haut et présentant un angle solide de 30° à 90°, la surface périphérique étant placée entre la périphérie de la surface conique et la paroi intérieure de la zone de mélange;

(b) établissement à l'intérieur de la zone de mélange de circuits d'écoulement coaxiaux concentriques qui sont maintenus physiquement séparés l'un de l'autre mais en communication d'écoulement de fluide les uns avec les autres à leurs extrémités supérieure et inférieure, le rapport du diamètre de la zone de mélange au diamètre du circuit d'écoulement intérieur étant de 2:1 à 5:1; et

(c) introduction dans un circuit d'écoulement extérieur parmi les circuits d'écoulement coaxiaux d'au moins un gaz sous forme de bulles en provenance de la surface périphérique, de manière à amener la phase liquide continue à s'écouler tout d'abord vers le haut dans ce circuit, puis à s'écouler vers le bas à travers le circuit d'écoulement intérieur parmi les circuits d'écoulement coaxiaux en direction de l'élément de fermeture d'extrémité inférieur et à revenir dans le circuit d'écoulement extérieur tout en maintenant la phase solide en suspension.

2. Procédé selon la revendication 1, caractérisé en ce que l'on introduit au moins un gaz supplémentaire sous forme de bulles dans le circuit d'écoulement extérieur, transversalement vers l'extérieur de ce dernier.

3. Procédé selon la revendication 1 ou 2, caractérisé en ce que la phase liquide contenue est soumise à une force propulsive auxiliaire en direction du bas dans le circuit d'écoulement intérieur.

4. Procédé selon l'une quelconque des revendications 1 à 3, caractérisé en ce que le gaz comprend de l'air, la phase liquide continue comprend un milieu de fermentation, et les matières solides en particules comprennent un organisme et un substrate solide pour cet organisme.

5. Procédé selon la revendication 4, caractérisé en ce que les matières solides en particules comprennant une matière contenant de la cellulose et l'organisme est un organisme qui provoque la lyse de la cellulose.

6. Appareil pour effectuer un contact multiphase entre des phases gazeuse, solide et liquide, caractérisé par

(a) un récipient cylindrique vertical présentant un rapport de hauteur à diamètre de 1:1 à 4:1;

(b) une surface conique présentant un angle solide de 30° à 90° s'étendant vers le haut à l'intérieur du récipient depuis l'extrémité inférieure et dont le pourtour est espacé de la paroi du récipient vers l'intérieur;

(c) un dispositif de production de bulles de gaz placé à l'extrémité inférieure du récipient et dans l'intervalle compris entre la paroi intérieure et la périphérie de la surface conique pour introduire au moins un gaz sous forme de bulles dans une phase liquide continue contenue dans le récipient et dans laquelle se trouve en suspension une phase solide formée de particules; et

(d) un tube d'aspiration cylindrique placé à l'intérieur du récipient coaxialment à ce dernier avec son extrémité inférieure espacée vers le haut par rapport au sommet de la surface conique et avec son extrémité supérieure espacée vers le bas par rapport au niveau prévu du liquide dans le récipient, le rapport du diamètre du récipient au diamètre du tube d'aspiration étant de 2:1 à 5:1.

7. Appareil selon la revendication 6, caractérisé en ce que le dispositif de production de bulles de gaz comprend une pluralité d'orifices d'un diamètre des 0,05 à 1 cm formés à travers l'élément de fermeture inférieur au voisinage de la paroi du récipient et espacés circonférentiellement du façon équidistante les uns des autres de 1 à 10 cm.

8. Appareil selon la revendication 6 ou 7, caractérisé en ce que le tube d'aspiration est placé à 5—10 cm au-dessus du sommet de la surface conique et à 5—10 cm en-dessous du niveau prévu du liquide dans le récipient.

9. Appareil selon l'une quelconque des revendications 6 à 8, caractérisé en ce qu'on dispositif auxiliaire de production de bulles de gaz entoure le tube d'aspiration et est réalisé de mainère à expulser dans la phase liquide du gaz sous forme de bulles radialement vers l'extérieur du tube.

10. Appareil selon l'une quelconque des revendications 6 à 9, caractérisé en ce qu'une roue à ailette de propulsion de liquide vers le bas est disposée en un point adjacent à l'extrémité supérieure du tube d'aspiration.

FIG.1.

FIG. 2.